# EUROPÄISCHE PATENTSCHRIFT

(11) **EP 1 785 117 B1**
(45) Veröffentlichungstag und Bekanntmachung des Hinweises auf die Patenterteilung: **15.10.2008**
(21) Anmeldenummer: 05024778.2
(22) Anmeldetag: 14.11.2005
(51) Int. Cl.: A61G 13/00, A61G 12/00, A61B 19/02

(54) **Konsole mit einem Stauraum**
Console with storing place
Console avec espace de rangement

(43) Veröffentlichungstag der Anmeldung: 16.05.2007
(73) Patentinhaber: Ondal Holding GmbH, 36088 Hünfeld (DE)
(72) Erfinder: Knappe, Stefan, 36151 Burghaun (DE); Schlitt, Jürgen, 36272 Niederaula (DE)
(74) Vertreter: Graf von Stosch, Andreas

(56) Entgegenhaltungen:
- DE-A1- 3 923 316
- DE-U1- 29 702 778
- US-A- 5 452 807
- US-B1- 6 497 075

## Beschreibung

Es ist bekannt, im Klinikbereich Konsolen mit Tabletts zum Tragen von medizinischen Geräten zu nutzen. Dabei werden die Geräte auf den Tabletts angeordnet. Die elektrischen Anschlussleitungen der Geräte werden über die Tabletts gelegt und die Stecker der Anschlussleitung werden in Steckdosen gesteckt. In analoger Weise passiert dies mit Gasleitungen, Die Tabletts sind einfach aufgebaut und werden entweder mittels eines Stecksystems auf eine bestimmte, vorgegebene Höhe gesteckt oder mittels einer relativ aufwändigen Montageeinrichtung auf einer bestimmten Höhe fixiert.

Die bekannten Konsolen haben den Nachteil, dass die Kabel bzw. Leitungen manchmal zu lang sind und die überschüssigen Kabel- bzw. Leitungslängen Personen hindern oder sogar Stolperfallen darstellen können.

Ferner offenbart die Deutsche Patentanmeldung DE 39 23 316 A1 einen Arbeitstisch für die Bildung eines Bildschirm-Arbeitsplatzes. Der Arbeitstisch umfasst eine Tischplatte und Seitenständer zum Tragen der Tischplatte. Unterhalb der Tischplatte ist eine Wanne vorgesehen. Die Wanne dient der Aufnahme elektrischer Anschluss- und/oder Verteilergeräte oder Kabel. Eine Zugangsöffnung ist in der Tischplatte vorgesehen, durch die auf den Wanneninnenraum zugegriffen werden kann.

Nachteilig an den bekannten Konsolen ist ferner, dass sie wenig flexibel einsetzbar sind. Die Höhe des Tabletts bzw. Arbeitstisches soll möglichst einfach und flexibel wählbar sein.

Es liegt die Aufgabe zugrunde, diese Nachteile zu beseitigen.

Gelöst ist die Aufgabe gemäß Anspruch 1. Danach weist die Konsole eine Montageeinrichtung auf, an welcher mindestens ein Tablett an einer Trageeinrichtung befestigt ist. Das Tablett weist eine obere Wandung und eine untere Wandung auf. Zwischen den Wandungen ist mindestens ein Stauraum vorgesehen, welcher zur Aufnahme eines Elektrokabels, vorzugsweise mitsamt einem Netzstecker, oder eines sonstigen Kabels bzw. einer Versorgungsleitung vorgesehen ist. Zwei vertikale Tragrohre sind vorgesehen und mindestens zwei Montageklauen, wobei die Montageklauen derart an den Tragrohren befestigt sind, dass zwei Montageklauen nebeneinander an den Tragrohren vorgesehen sind, und wobei an den Montageklauen jeweils eine Steckverbindung vorgesehen ist, und ist mindestens ein Tablett vorgesehen, welches zwei Aufnahmen zum jeweiligen Aufnehmen einer Steckverbindung aufweist, um derart das Tablett auf die beiden Montageklauen stecken zu können.

Die vorgeschlagene Neuerung hat den Vorteil, dass irgendeine Leitung oder irgendein Kabel zumindest teilweise innerhalb eines Tabletts aufbewahrt werden kann, da das Tablett eine obere Wandung und eine untere Wandung aufweist, und die Aufnahmen zwischen den beiden Wandungen vorgesehen sind. Es werden die Räume zwischen den Wandungen als Stauraum genutzt. Ein derartiger Stauraum wird zur Aufnahme eines Kabels oder einer beliebigen Versorgungsleitung (Gas, Wasser) genutzt. Damit wird erreicht, dass Leitungen bzgl. ihrer überschüssigen Länge sicher verwahrt werden. Stolperfallen durch Kabel oder Kabelschleifen werden somit vermieden. Das Tablett kann in einfacher Weise auf einer beliebigen Höhe fixiert werden. Die beiden Montageklauen, die das Tablett halten, können auf irgendeiner Höhe, die dem Zweck des auf dem Tablett befindlichen medizinischen Arrangements genügt, an den beiden Stangen montiert werden. Die Steckverbindungen werden in die Aufnahmen des Tabletts gesteckt. Somit ist die Montage des Tabletts durchgefühlt.

Vorteilhafte Ausgestaltungen der vorgeschlagenen Neuerung sind in den Ansprüchen 2 bis 4 beschrieben.

Ist gemäß Anspruch 2 in der oberen Wandung, angrenzend an einen Stauraum, mindestens eine Aussparung zum Durchfuhren eines mit einer Anschlussbuchse versehenen oder später zu versehenen Elektrokabels oder sonstigen Kabels (bzw. Leitung) vorgesehen, so kann ein durch einen Stauraum geführtes Kabel (außer einem Elektrokabel auch eine Wasserversorgung oder eine Gasversorgung) ein auf dem Tablett befindliches Gerät versorgen. Am Tablett ist dabei nur das Kabelstück, welches von der Aussparung bis zum Gerät reicht, zu sehen. Mehrere Aussparungen dienen zum Durchführen mehrerer Kabel, Datenleitung oder ähnlichem.

Ist an der Konsole ein Korpus vorgesehen, der eine Mehrzahl an Steckdosen oder Versorgungen für Gas oder Wasser aufweist, die über eine Versorgungsleitung mit elektrischem Strom, Gas oder Wasser versorgt werden (Anspruch 3), so kann für die Kabel, die sich in den Stauräumen befinden, in kurzer Entfernung zu einem Tablett eine Versorgung erfolgen, ohne dass zu lange Kabelstrecken stören. Die Versorgungsleitung wird vorzugsweise von oben her zur Konsole geführt. Dies ist besonders vorteilhaft, da in einer bevorzugten Ausführungsform die Tragrohre an einem Deckenstativ hängen sollten.

Die Konsole könnte auch in einem Friseursalon eingesetzt werden und dort lediglich für eine Elektroversorgung ausgelegt sein.

Sind die Aufnahmen zwischen den Wandungen vorgesehen (Anspruch 4), so wirken sie stabilisierend für das Tablett.

Im Folgenden wird die Erfindung an Hand ein Ausführungsbeispiel darstellender Figuren näher beschrieben. Es zeigt:

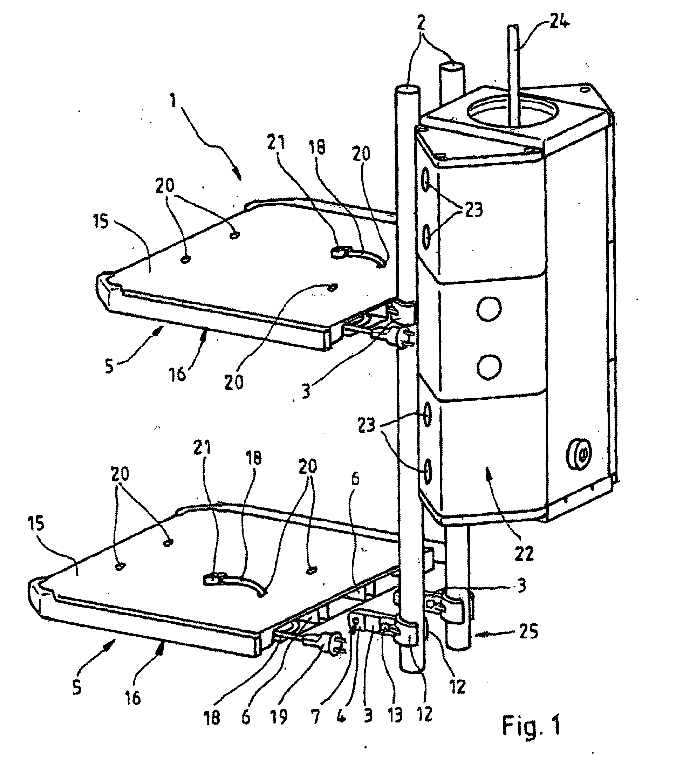

Figur 1 in einer perspektivischen Ansicht eine Konsole mit zwei vertikalen Tragrohren und zwei Tabletts, in denen jeweils Stauräume zum Aufnehmen von Elektrokabeln vorgesehen sind, und die mittels jeweils zweier Steckverbindungen an den Tragrohren befestigt werden;

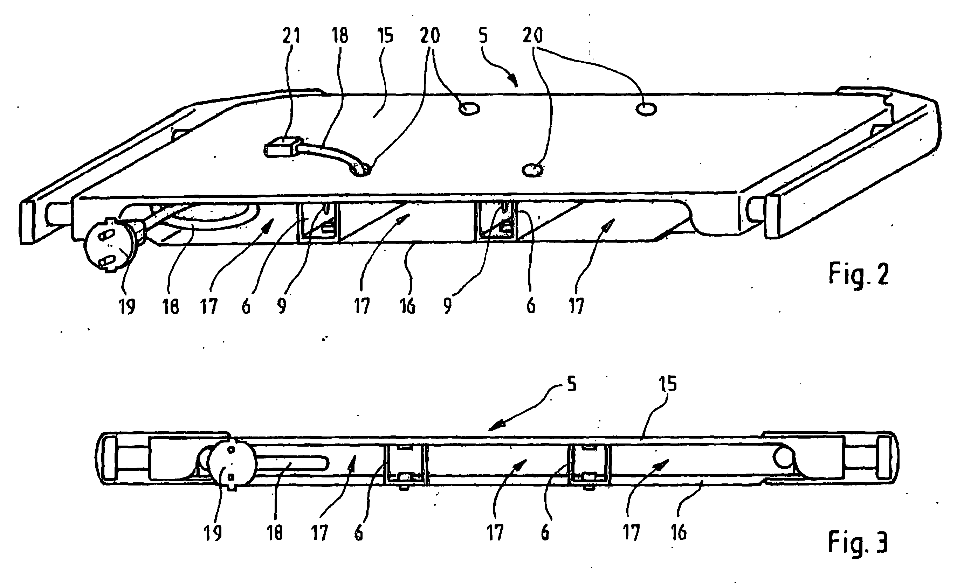

Figur 2 in einer perspektivischen Ansicht ein Tablett der Figur 1;
Figur 3 in einer Seitenansicht das Tablett der Figur 2;

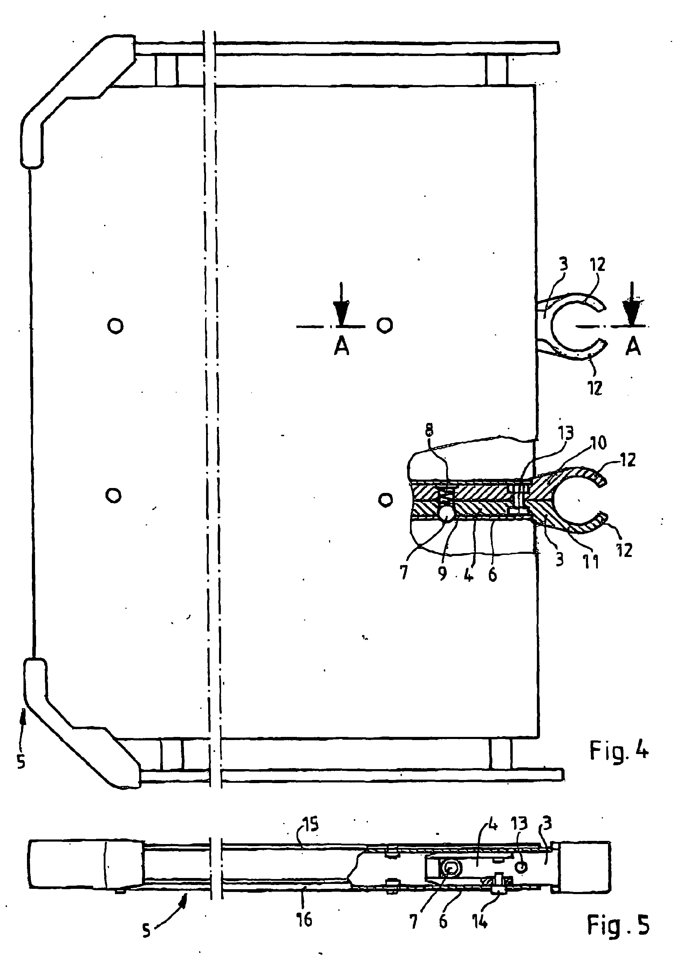

Figur 4 in einer Ansicht von oben mit teilweisem Schnitt das Tablett der Figur 2 jedoch ohne Elektrokabel und mit eingesteckten Steckverbindungen, sowie
Figur 5 in einer um 90 Grad zur Ansicht der Figur 3 versetzten Seitenansicht mit teilweisem Schnitt das Tablett der Figur 4.

Bei einer Konsole 1 für den Klinikbereich, mit zwei vertikalen Tragrohren 2, und mit vier Montageklauen 3, sind die Montageklauen 3 derart an den Tragrohren 2 befestigt, dass jeweils zwei Montageklauen 3 nebeneinander an den Tragrohren 2 vorgesehen sind. An den Montageklauen 3 ist jeweils eine Steckverbindung 4 ausgebildet. An jedem von zwei. Tabletts sind jeweils zwei als Rechteckrohr ausgeführten Aufnahmen 6 zum jeweiligen Aufnehmen einer Steckverbindung 4 vorgesehen, um derart das Tablett auf die beiden Montageklauen 3 stecken zu können.

Die Steckverbindung 4 weist eine Kugel 7 auf, die in der Steckverbindung 4 gehalten wird, die mittels einer Feder 8 gegenüber der Steckverbindung 4 vorgespannt ist. Die Kugel 7 ist zum Einrasten in eine Aussparung 9 der Aufnahme 6 vorgesehen.

Die Montageklaue 3 besteht aus zwei Klauenteilen 10, 11, die jeweils ein Greifelement 12 aufweisen. Die Klauenteile 10,11 sind mittels eines Verbindungselementes 13, welches als Schraube ausgeführt ist, miteinander verbunden. Ein Sicherungselement 14, welches eine Schraube ist, ist zum Sichern der Steckverbindung 4 in der Aufnahme 6 vorgesehen.

Jedes Tablett 5 weist eine obere Wandung 15 und eine untere Wandung 16 auf. Es sind jeweils zwei Aufnahmen 6 und drei Stauräume 17 zwischen den beiden Wandungen 15, 16 eines Tabletts vorgesehen. Die Stauräume 17 sind zur Aufnahme von Elektrokabeln 18 mitsamt deren Netzstecker 19 vorgesehen. Dabei wird der Netzstecker 19 in den Stauraum 17 eingeklemmt. Der Wandungsabstand ist entsprechend bemessen. In der oberen Wandung 15, angrenzend an den linken und den mittleren Stauraum 17 sind insgesamt vier Aussparungen 20 zum jeweiligen Durchführen eines später mit einer Anschlussbuchse 21 zu versehenen Elektrokabels 18 vorgesehen. Die Anschlussbuchse 21 dient zum Anschließen eines medizinischen Gerätes. An der Konsole 1 ist ein Korpus 22 vorgesehen, der eine Mehrzahl an Steckdosen 23 aufweist. Die Steckdosen 23 werden über eine elektrische Versorgungsleitung 24 mit elektrischen Strom versorgt.
- 1: Konsole
- 2: Tragrohr
- 3: Montageklaue
- 4: Steckverbindung
- 5: Tablett
- 6: Aufnahme
- 7: Kugel
- 8: Feder
- 9: Aussparung
- 10, 11: Klauenteil
- 12: Greifelement
- 13: Verbindungselement
- 14: Sicherungselement
- 15, 16: Wandung
- 17: Stauraum
- 18: Elektrokabel
- 19: Netzstecker
- 20: Aussparung
- 21: Anschlussbuchse
- 22: Korpus
- 23: Steckdose
- 24: Versorgungsleitung
- 25: Montageeinrichtung

## Patentansprüche

1. Konsole (1), insbesondere für den Klinikbereich, mit einer Trageeinrichtung, die insbesondere zwei vertikale Tragrohre (2) aufweist, sowie einer Montageeinrichtung (25), mittels welcher mindestens ein Tablett (5) an der Trageeinrichtung befestigt ist, wobei das Tablett (5) eine obere Wandung (15) und eine untere Wandung (16) aufweist, und zwischen den Wandungen (15, 16) mindestens ein Stauraum (17) vorgesehen ist, welcher zur Aufnahme eines Elektrokabels (18), vorzugsweise mitsamt eines Netzsteckers (19), oder eines sonstigen Kabels vorgesehen ist,
**dadurch gekennzeichnet, dass**
zwei vertikale Tragrohre (2) vorgesehen sind, und mindestens zwei Montageklauen (3), wobei die Montageklauen (3) derart an den Tragrohren (2) befestigt sind, dass zwei Montageklauen (3) nebeneinander an den Tragrohren (2) vorgesehen sind, und wobei an den Montageklauen (3) jeweils eine Steckverbindung (4) vorgesehen ist, sowie mit mindestens einem Tablett (5), welches zwei Aufnahmen (6) zum jeweiligen Aufnehmen einer Steckverbindung (4) aufweist, um derart das Tablett (5) auf die beiden Montageklauen (3) stecken zu können.

2. Konsole nach Anspruch 1, **dadurch gekennzeichnet, dass** in der oberen Wandung (15), angrenzend an einen Stauraum (17), mindestens eine Aussparung (20) zum Durchführen eines Elektrokabels (18) oder sonstigen Kabels vorgesehen ist, welches vorzugsweise mit einer Anschlussbuchse (2i) versehen ist

3. Konsole nach Anspruch 1 oder Anspruch 2, **dadurch gekennzeichnet, dass** an der Konsole (1) ein Korpus (22) vorgesehen ist, der eine Mehrzahl an Steckdosen (23), Wasserversorgungen oder Gasversorgungen aufweist, die über eine elektrische Versorgungsleitung (24) mit elektrischen Strom bzw. über eine Versorgungsleitung mit Gas oder Wasser versorgt werden.

4. Konsole nach mindestens einem der Ansprüche 1 bis 3, **dadurch gekennzeichnet, dass** die Aufnahmen (6) zwischen den beiden Wandungen (15, 16) vorgesehen sind.

## Claims

1. Console (1), particularly for the hospital sector, comprising a support device which includes in particular two vertical support tubes (2), and a mounting device (25), by means of which at least one tray (5) is secured on the support device, wherein the tray (5) includes an upper wall (15) and a lower wall (16), and at least one storage space (17) is provided between the walls (15, 16), which is provided for receiving an electric cable (18), preferably together with a power plug (19), or other kind of cable,
**characterized in that**
two vertical support tubes (2) and at least two mounting claws (3) are provided, wherein the mounting claws (3) are secured on the support tubes (2) in such a manner that two mounting claws (3) are provided side-by-side on the support tubes (2), and wherein a plug connection (4) is provided on each of the mounting claws (3), and comprising at least one tray (5), which has two receptacles (6) for the accommodation in each case of a plug connection (4) thereby to be able to insert the tray (5) onto both of the mounting claws (3).

2. Console according to claim 1, **characterized in that** at least one recess (20) is provided in the upper wall (15) bordering on the storage space (17) for routing of an electric cable (18) or other cable, which is preferably provided with a connecting socket (2i).

3. Console according to claim 1 or claim 2, **characterized in that** a body (22) is provided on the console (1), which body comprises a plurality of socket-outlets, water supplies or gas supplies, which are supplied with electric current via an electric supply line (24) or, respectively, with gas or water via a supply line.

4. Console according to at least one of the claims 1 to 3, **characterized in that** the receptacles (6) are provided between the two walls (15, 16).

## Revendications

1. Console (1), en particulier pour le secteur clinique, comprenant un dispositif porteur, qui présente en particulier deux tuyaux porteurs verticaux (2), et un dispositif de montage (25), au moyen duquel au moins une tablette (5) est fixée sur le dispositif porteur, la tablette (5) présentant une paroi (15) supérieure et une paroi (16) inférieure, et au moins un espace de rangement (17) étant prévu entre les parois (15, 16), lequel espace est prévu pour le logement d'un câble électrique (18), de préférence avec une fiche de secteur (19), ou d'un autre câble,
**caractérisée en ce que**,
deux tuyaux porteurs (2) verticaux sont prévus, et au moins deux bras de montage (3), les bras de montage (3) étant fixées sur les tuyaux porteurs (2) de telle sorte que deux bras de montage (3) sont prévues l'une à côté de l'autre sur les tuyaux porteurs (2), et à chaque fois un connecteur enfichable (4) est prévue sur les bras de montage (3), et avec au moins une tablette (5), qui présente deux logements (6) pour le logement respectif d'une liaison à enfichage (4), afin de pouvoir enficher ainsi la tablette (5) sur les deux bras de montage (3).

2. Console selon la revendication 1, **caractérisée en ce qu'**un évidement (20) pour le passage d'un câble électrique (18) ou d'un autre câble est prévu dans la paroi (15) supérieure, de façon contiguë à un espace de rangement (17), lequel câble est doté d'au moins une douille de raccordement (21).

3. Console selon la revendication 1 ou la revendication 2, **caractérisée en ce que** sur la console (1) est prévu un corps (22) qui présente une pluralité de prises (23), d'alimentations en eau ou d'alimentations en gaz, qui sont alimentées par une conduite d'alimentation électrique (24) en courant électrique respectivement par une conduite d'alimentation en gaz ou en eau.

4. Console selon au moins l'une quelconque des revendications 1 à 3, **caractérisée en ce que** les logements (6) sont prévus entre les deux parois (15, 16).
